# EUROPEAN PATENT SPECIFICATION

(11) **EP 4 204 077 B1**
(45) Date of publication and mention of the grant of the patent: **03.04.2024**
(21) Application number: 22734330.8
(22) Date of filing: 05.07.2022
(51) Int. Cl.: A61N 1/36, A61N 1/04

(54) **COMPUTER-IMPLEMENTED METHOD FOR ENABLING PATIENT-SPECIFIC ELECTROSTIMULATION OF NEURONAL TISSUE**
COMPUTERIMPLEMENTIERTES VERFAHREN ZUR ERMÖGLICHUNG EINER PATIENTENSPEZIFISCHEN ELEKTROSTIMULATION
PROCÉDÉ MIS EN OEUVRE PAR ORDINATEUR POUR PERMETTRE UNE ÉLECTROSTIMULATION SPÉCIFIQUE À UN PATIENT D'UN TISSU NEURONAL

(30) Priority: 19.07.2021 EP 21186483; 27.05.2022 EP 22175856
(43) Date of publication of application: 05.07.2023
(73) Proprietor: Bottneuro AG, 4056 Basel (CH)
(72) Inventor: OSMANI, Bekim, 4056 Basel (CH); TÖPPER, Tino, 79115 Freiburg (DE); JOODAKI, Mahyar, 4051 Basel (CH)
(74) Representative: Mertzlufft-Paufler, Cornelius
(86) International application number: PCT/EP2022/068626
(87) International publication number: WO 2023/001546

(56) References cited:
- US-A1- 2012 265 261
- US-A1- 2017 165 485
- US-A1- 2017 312 517
- US-A1- 2021 196 943

## Description

The present disclosure is related to the field of electrical stimulation of neuronal tissue for treatment of neurodegenerative diseases, in particular treatment of Alzheimer's disease (AD).

In particular, the invention concerns a computer-implemented method for computing a patient-specific set of configuration parameters suitable for configuring and/or choosing an electrical stimulation device that is designed for electrical stimulation and/or recording of neuronal tissue, in particular of cerebral tissue. Using this method, a patient-specific electrical stimulation of the patient's neuronal tissue is possible by choosing and or configuring the stimulation device according to the derived patient-specific set of configuration parameters. The invention also concerns a method for computing a series of individual sets of such optimized configuration parameters.

Furthermore, the invention concerns a computer program for implementing the method.

It is important to note here that the method presented herein neither includes a step of treating the human or animal body by surgery or therapy and also no diagnostic step practiced on the human or animal body; rather the invention is concerned with benefiting from pre-existing diagnostic data sets (e.g., MRT- or PET-images) and using them for configuring an electrical stimulation device in a "best-case" effort. After the method according to the invention has been performed, the configured device may be used by any person wishing to use it for efficient electro-therapy. This electrostimulation-therapy may be even combined with auditory and/or visual stimulation. It is also to be stressed, that the method proposed here does not produce electrical field distributions in reality but rather virtually within a computer simulation. This avoids time-consuming and costly trial-and-error manual configuration of a stimulation device. Instead, the best-case configuration can be computed fully computer-implemented and standardized, without significant intervention from a practitioner. We also note that simultaneous Electroencephalography (EEG) recording and electrostimulation is possible in case the generated electrical fields are localized and stimulation noise artifacts can be removed from the EEG recording.

The method presented herein is particularly relevant to the treatment of Alzheimer's disease (AD), which is the 6th leading cause of death in the United States, with more than 5 million people affected by AD in the United States alone. Although the causes of AD are yet to be fully understood, this neurodegenerative disease is largely associated with amyloid plaques, neurofibrillary tangles, and loss of neuronal connections in the brain. AD results in a loss of cognitive, emotional and social abilities of the patient and causes immense costs within the health-care-system, with a strong growth in recent years, due to the aging populations in developed countries.

Amyloid plaques are extracellular deposits of the amyloid beta (Aβ) protein mainly in the grey matter of the brain and are very often referred to as "debris". Current treatments of AD focus on clearing the brain from these plaques or debris. However, latest clinical studies have shed severe doubts on the effectiveness of this therapeutic approach.

Recently, it has been found that 40 Hz acoustic stimulation decreases amyloid beta deposition in the brain in certain patients (cf. Lee, J. et al: "40 Hz acoustic stimulation decreases amyloid beta and modulates brain rhythms in a mouse model of Alzheimer's disease" bioRxiv 2018).

Another important approach for treating AD is the electrical stimulation of glial cells (or neuroglia), in particular of microglia, which are non-neuronal cells in the central nervous system (brain and spinal cord) and the peripheral nervous system that - different from neurons - do not produce electrical impulses. Microglia are a type of neuroglia (glial cells) located throughout the brain and spinal cord. Microglia are resident macrophage cells, which act as the first and main form of active immune defense in the central nervous system and have been found to be highly important to the development of AD. Neurons (or nerve cells), on the other hand are electrically excitable cells that communicate with other cells via specialized electro-chemical connections called synapses. Neurons are the main component of the nervous tissue, in particular in the brain.

This approach is sometimes referred to as "low intensity transcranial electric stimulation" because the electrical fields are typically produced outside of the brain using non-invasive stimulation electrodes and thus transferred into the brain via the skull bones (thus "transcranial"). It has not only been applied to the treatment of AD but also to mental diseases such as major depression or for treating neuropathic pain as a result of nerve injuries or tinnitus, Parkinson's disease, post-stroke aphasia. By modulating axonal membrane potential, transcranial current stimulation (tCS) can interact with various endogenous neural network features, such as ion channel dynamics, spike timing, firing rate, oscillatory potential, synaptic transmission, and also brain responses to external stimuli.

A typical DC-stimulation scheme (tDCS = transcranial DC stimulation) employs a bipolar montage, in which there is at least one negatively charged cathode and at least one positively charged anode; however, AC-stimulation schemes (tACS = transcranial AC stimulation) have also been applied in the past.

For example, US 2012 265261 A1 deals with neurocranial electrostimulation and discloses for this purpose in Figure 4 a cap (skull cap 430) with an arrangement of electrodes 310, with which the brain of a patient can be electrically stimulated using a DC-stimulation scheme.

US 2017 312517 A1 discloses an approach for recording of electrical brain signals in the context of "training and assessment" of a person's behavioral performance. For this purpose, the subject is subjected to neurological (but not electrical) stimulation while the subject performs specified tasks.

Current methods are, however, limited in their effectiveness, because tCS typically results in an insufficient focusing of the currents injected / the electrical fields produced in the brain. Therefore, multifocal tCS has been developed using a number of non-invasive stimulation electrodes. It has also been found that the duration of electrostimulation, both on large and small timescale, and also the duration and repetition of an individual stimulation session play important roles for the success of the therapy.

Furthermore, gamma frequency stimulation, which is a specific type of AC electrostimulation in a frequency range of between 35..100 Hz, has been proposed for treatment of AD (cf. Martorell, A.J. et al. "Multi-sensory Gamma Stimulation Ameliorates Alzheimer's-Associated Pathology and Improves Cognition". Cell (2019))

In this context, one objective of the invention is to provide means which allow patient-specific and hence effective AC electrostimulation of neuronal tissue, in particular in the brain. In particular, the invention aims at a method that can rapidly deliver a best-case configuration of an electrostimulation to be later applied to a patient. A particular desire is that the means should enable a largely autonomous therapy by transcranial alternating current stimulation (tACS), which is a subtype of AC electrostimulation different from transcranial direct current stimulation (tDCS), the latter being a DC electrostimulation. The goal is that this novel tACS-therapy can be performed by the patient himself, remote from a hospital. The approach of the invention, however, is not only applicable to the treatment of neurodegenerative diseases such as AD but also to other sorts of electrostimulation of neuronal tissue, also beyond therapeutic aims.

In accordance with the present invention, a method is provided according to claim 1, which solves the afore-mentioned problem. In particular, the invention proposes an (at least partly) computer-implemented method as introduced at the beginning, which, in addition, may be characterized in that the method comprises the following steps:
A) extracting patient-specific data including at least one region of interest (ROI) from at least one pre-existing image (3) previously acquired using a medical imaging technique; of course, these images should show regions of the brain of the patient in which the electrostimulation is to be applied later.
B) computer simulation of an electrical field distribution that can be generated using the electrical stimulation device (1) when configured with a specific set of configuration parameters defining a specific transcranial alternating current stimulation (tACS) scheme, taking into consideration the extracted patient-specific data;
C) Variation of a set of simulation parameters during iterative application of step B) to compute the patient-specific set of configuration parameters, which are thus optimized for the patient; and
D) configuring the stimulation device (1) with the derived set of configuration parameters such that the stimulation device (1) is configured to focus the electrostimulation onto the at least one region of interest (ROI).

In other words, the method may be implemented by:
producing a numerical and/or geometrical model of the patients neuronal tissue to be electro-stimulated;
performing computational modeling of electrostimulations possible with the device after it has been configured and/or chosen according to a specific set of configuration parameters;
and finally identification of an optimized set of these configuration parameters (which may include the choice of a particular stimulation device comprised in a limited set of M available stimulation devices), which - if applied to the chosen / present stimulation device, will lead to a patient-specific electrostimulation that is optimized for the needs of the patient, i.e. the electrostimulation is optimally focused to the at least one region of interest (ROI) extracted from the at least one pre-existing image.

Although the computational effort may be large for implementing the method proposed herein, cloud computing may be employed to rapidly deliver the desired set of configuration parameters optimized for the individual patient. We also note that, different from tDCS, where the total field strength or current inside the brain can be simply calculated as a superposition of the contribution of each DC-electrode, the computing of field strength in an tACS-scheme is much more complex, as the relative phases of the individual fields generated by each one of the stimulation electrodes employed has to be taken into account, because the final field strength will be the result of constructive and destructive interference of electromagnetic waves. One major advantage of using tACS is that smaller ROI (also deeper in the brain) can be targeted using more localized electric fields, as compared to tDCS.

We further note at this point, that the method proposed herein is particularly useful for multi-focal tACS, as the configuration of the stimulation device can be such that the electrostimulation (i.e., the maximum field strength) is focused on several different ROI (which have been extracted from the images and which are located inside the brain of the patient). In other words, the computed electrical field distribution may show several maxima of the field strength, each located at a specific ROI. For example, the fields may be maximized locally in each of the (or at least some of the) specific multiple ROI that have been identified in the pre-existing images of the patient's head. This can be achieved, for example, by constructive interference of the applied electrical fields in at least two different ROI.

The extraction of the ROI(s) from the pre-existing images can be performed or at least monitored by a clinician. However, as the performance of artificial intelligence (AI) is increasing, the extraction of the ROI(s) may be fully automated and computer implemented, i.e., without human intervention.

A bit simplified, the major aim of the method is to derive the configuration parameters necessary for achieving an electrostimulation with the stimulation device that represents a best-case scenario, given typical boundary conditions such as the available number of stimulation electrodes, their physical shape and/or geometrical arrangement as well as other limiting factors such as current and frequency range available for the driving currents to be applied to the individual electrodes.

Most conveniently, the simulation parameters and/or the final set of configuration parameters may be processed as a digital data set, respectively. In addition, the simulation parameters may comprise (in some cases, they may be identical to) the configuration parameters.

Step B may be performed according to an optimization method, in particular including a FEM-method for calculating the E-field distributions. The goal of the optimization can be maximizing an electrical field strength in at least one region of interest, in particular while minimizing the electrical field strength elsewhere.

The variation of the simulation parameters will typically comprise a variation of said configuration parameters, for example (virtually) moving a location of one of the N electrical stimulation electrodes or (virtually) adapting an 3D-arrangement of the N stimulation electrodes of the device.

To provide one possible application case, it is well known, that the activity of glial cells can be modulated by electrical stimulation. In response to electrical fields of a well-defined field strength and frequency, these cells can be stimulated to secrete anti-inflammatory agents (e.g., IGF1, TGFB, IL-10). One particularly suitable approach is the electrical stimulation to inhibit the release of pro-inflammatory cytokines and to promote the release of anti-inflammatory cytokines und insulin degrading enzymes (IDE). By this approach, the glial cells can be transferred from a so-called M1 state, in which there is an abundance of pro-inflammatory cytokines, which are prominent in AD, to a so-called M2 state, in which there is a surplus of anti-inflammatory, debris clearing cytokines.

In the brain tissue, gray matter is distinguished from white matter: gray matter is a major component of the central nervous system, consisting of neuronal cell bodies, neuropil (dendrites and unmyelinated axons), glial cells (astrocytes and oligodendrocytes), synapses, and capillaries. Gray matter is distinguished from white matter in that it contains numerous cell bodies and relatively few myelinated axons, while white matter contains relatively few cell bodies and is composed chiefly of long-range myelinated axons. The color difference arises mainly from the whiteness of the myelin.

The proposed extraction of patient-specific data during step A) of the method can include such information, in particular the distribution of gray and white matter within the brain, and thereby also a local distribution of glial cells. Hence, such patient-specific data allows the (preferably computer-implemented) identification of at least one, i.e., in particular several, region(s) of interest (ROI) inside the brain to be electro-stimulated. The task to be solved during steps B) and C) of the method is thus to derive the set of configuration parameters such that a focused (i.e., localized) electrostimulation of the identified at least one ROI is possible, after the electrical stimulation device has been configured with or at least chosen (from a set of available devices) based on said optimized configuration parameters.

The patient-specific data extracted, preferably by a computer implemented algorithm, from the at least one image, can also be fed to a computational module, which computes a patient-specific head/skull model from that data. This head model can include several layers (representing spinal cord, grey matter, white matter, bones, scalp, etc.). Each layer may be specified in its electrical properties (conductivity, capacitance, impedance etc.), in particular based on pre-existing data (e.g., tabulated electrical data of various human tissues and other anatomical structures) and/or with respect to frequency (e.g., the impedance depends on the electrical frequency). Such a head model can thus form the basis of the computer simulations to be performed during step B), as it allows accurate modeling of the electrical properties of the head of the individual patient and thus the interaction with the external electrical fields produced with the stimulation electrodes.

The computational modeling / simulation performed in step B) aims at computing a number of electrical field distributions, which - in principal - could be generated, for example using either a fixed set of N stimulation electrodes that can be freely arranged (i.e. using a stimulation device with a configurable electrode arrangement) or a given set of N stimulation electrodes arranged in a fixed (or only slightly adaptable) arrangement. Depending on the application, the invention suggests to limit the number of electrodes to 32, or even 16 or even only 10 electrodes. This way, the computational effort can be reduced and such numbers can still be sufficient to provide a high spatial resolution of the tCS within a limited region of the brain.

As explained previously, the method may aim at enabling focused electrostimulation in at least one identified region of interest (ROI). For this purpose, it is important not to stimulate tissue regions outside of this ROI. This may be achieved by configuring the stimulation device such that regions outside of the ROI only receive low magnitudes of the electrical field (and resulting injected currents) generated with the device. For enabling a best-possible focused electrostimulation, a sub-critical electrostimulation of the regions out of the ROI is proposed. "Sub-critical electrostimulation" may be achieved, by making provisions that the electrical fields remain below an action potential (AP) of neuronal cells present in the ROI. For example, the AP of typical neuronal cells in the brain is in the order of 50 mV. Considering the typical size of a neuron of 20 to 30 µm, this results in an upper limit for the electrical field strength to be applied in the order of 2.0 kV/m. Therefore, the computer simulation may be directed at safeguarding such "sub-critical electrostimulations" in tissues regions outside the ROI.

The method will now be described in greater detail: For example, step C) of the method may comprise a variation of a physical configuration of N stimulation electrodes of the stimulation device. In particular, the variation may comprise a change of a location of one of the N stimulation electrodes or a size and/or shape of one of the N stimulation electrodes.

It is also understood that N stimulation electrodes of the stimulation device may be used later on during therapy to generate one optimized electrical field distribution computed in the simulation. Hence the electrical field distribution can be generated with the N stimulation electrodes.

The set of patient-specific configuration parameters derived with the method may comprise at least one, preferably at least two, of the following parameters:
a specific geometrical arrangement of N stimulation electrodes of the stimulation device achievable by re-arranging the electrodes within a limited geometrical adjustment range offered by the stimulation device (for example, the stimulation device may be designed as a head bonnet with the stimulation electrodes movable on the bonnet within a limited xy-adjustment range);
a specific geometrical arrangement of N stimulation electrodes belonging to one particular stimulation device comprised in a limited number M of stimulation devices each featuring a pre-defined, in particular non-reconfigurable, respective arrangement of the N stimulation electrodes (Hence the result of the optimization may be a choice of one particular of the M stimulation devices);
a relative distribution of N driving currents or driving voltages to be applied to N stimulation electrodes of the stimulation device (hence a certain electrical driving scheme may be part of the configuration parameters);
a relative distribution of phase lags of N driving AC currents or N driving AC voltages to be applied to N stimulation electrodes of the stimulation device;
a timing-scheme for N driving AC voltages to be applied to N stimulation electrodes of the stimulation device, in particular including N specific relative time-lags and/or a respective time sequence of activation of each of the stimulation electrodes;
a set of N waveforms of N driving AC voltages to be applied to N stimulation electrodes of the stimulation device;
It is understood here that technically, controlling driving voltages or controlling driving currents is equivalent.

Concerning the specific waveform of one of the N voltages to be applied to the respective stimulation electrode, the waveform may be based, for example, on a square wave signal with a superimposed higher-frequency signal component. Depending on the application, other waveforms such a triangular signal forms may be used. In general, the voltage signal applied to one of the stimulation electrodes in a tACS-scheme may be a complex wavelet.

The derived configuration parameters can also comprise a patient-specific electrical stimulation protocol for driving a number of N stimulation electrodes of the device. In such a case, the stimulation protocol may comprise at least one, preferably at least two, of the following electrical stimulation parameters:
- amperage and/or
- frequency and/or
- ac/dc driving scheme and/or
- relative phase and/or
- waveform and/or
- relative time-lag
of individual driving currents to be applied to the individual stimulation electrodes of the stimulation device.

According to a preferred embodiment, the stimulation protocol may comprise at least three phases, to be applied during different time intervals and based on different base frequencies (e.g., f1 = 30Hz, f2 = 40 Hz, f3 = 50 Hz).

According to yet another variant of the method, which may be used in combination with other features presented herein, the electrical stimulation protocol comprised in the derived configuration parameters may define a tACS of at least one ROI using two different arrangements of m stimulation electrodes, wherein the electrodes of each arrangement are driven at different frequencies. Such a modeling can later result in a therapy in which excessive heating of the scalp and skin irritations are avoided, even when the electrostimulation is performed for several hours.

In addition, the method may further comprise a step of
D) configuring (either manually or computer-assisted or fully computer-implemented) the stimulation device with the derived set of configuration parameters, in particular with said electrical stimulation protocol.

In particular, the configuration of the stimulation device may be performed such that the stimulation device is configured to focus the electrostimulation onto at least one region of interest (ROI) that has been extracted from the at least one image during step A).

In other words, the stimulation device may be configured and/or chosen (in particular from a limited set of pre-configured stimulation devices, as described below) according to the set of configuration parameters, which was optimized during application of steps B) and C).

Furthermore, the method may also comprise (alternatively or additionally) a step of
E) choosing said electrical stimulation device out of a pre-defined set of M different stimulation devices based on the derived set of configuration parameters.

Following this approach, the M stimulation devices comprised in the pre-defined set may differ:
in their respective number N of available individual stimulation electrodes; and/or
in a respective, fixed or adjustable, geometrical arrangement of a number of N stimulation electrodes; and/or
in a respective individual shape of at least one of the respective stimulation electrodes.

In other words, each of said M stimulation devices may be pre-configured for producing a certain electrical field distribution.

For efficient therapy later on with the device, it is also beneficial if the patient-specific data extracted in step A) comprise at least one, preferably ate least two, of the following patient-specific parameters:
geometrical data of a skull of the patient, such as distribution of a thickness of the skull bone and/or of the scalp - such data are highly useful for computing an accurate head model, which can form the basis of the desired computer simulation of the electrical field distributions;
3D-distribution of brain tissue, in particular size of the patient's brain and/or distribution of gray and white matter within the brain of the patient - such parameters form important boundary conditions for the computer simulation;
localized concentrations of specific proteins, in particular of amyloid and/or tau proteins, within the brain of the patient - such data can be useful for defining the at least one ROI and can help to focus the electrical fields generated to ROI;
localized occurrence of atrophy in the brain of the patient - such data can be beneficial for fine-tuning the focus of the electrical fields to be generated;
3d-coordinates of at least one region of interest (ROI) to be electro-stimulated with the device (1);

In all of such cases, it may be preferable if the optimization performed in step B) takes into account at least one of the patient-specific parameters for computing said (optimized) set of configuration parameters, because this will help in configuring the stimulation device according to the specific needs of a particular patient.

Likewise, it will be beneficial, if the optimization performed in step B) is based on a geometrical head model derived from the patient-specific data extracted in step A), because this allows more accurate computer simulations.

According to a preferred embodiment, the at least one ROI, to which the electrostimulation is to be focused (using the patient-specific set of configuration parameters computed with the method), may be extracted/derived exclusively from pre-existing positron emission tomography (PET)-images, in particular without using magnetic resonance tomography (MRT)-images. PET-images are usually obtained by administering a weakly radioactive contrast medium (with a half-life of ca. 2h) to the patient and measuring the resulting positron emission. For example, depending on the contrast medium used, those areas inside the brain with low PET-signal may be identified as a ROI; or areas with high PET-signal may be identified as a ROI. The latter will be the case, if biomarkers such as tau proteins (which can indicate the presence of a localized inflammatory response) are used resulting in a so-called tau-PET-scan. A thresholding may be applied to differentiate between a ROI and tissues areas not to be electro-stimulated.

Most preferably, localized concentrations of specific proteins, in particular of amyloid and/or tau proteins, which are visible in the PET-images, may be used for defining the at least one ROI, which is then used as input for the computing. This approach is ideally suited for a cost-efficient patient-specific tCS-therapy for which the patient only needs to undergo a single PET-imaging session; the lower resolution of PET vs. MRT is still acceptable.

For example, a lesion segment's volume inside the brain may be identified as a ROI using a PET-scan. In such a case, the method may aim at maximizing the electrical field strength in that ROI. The frequency of the tACS interfering signals in the ROI can be equal to a frequency of the patient's Gamma wave. However, the frequency of the electrical tACS-signal applied to each electrode can be equal to or different from the patient's Gamma wave frequency, which is accessible by an Electroencephalography (EEG).

Furthermore, the method may aim at minimizing the E-field outside the ROI.

Likewise, in particular when using this approach, the mentioned head model may be derived exclusively from these PET-images. This approach thus suggests an MRT-free modeling of the brain. If MRT-images are already available, however, PET-images may be registered to a head model derived from these MRT-images (which typically offer higher spatial resolution than PET-images).

The simulation parameters varied in step C) may comprise at least one, preferably at least two, of the following parameters:
number N of available stimulation electrodes of the device;
geometrical arrangement of N available stimulation electrodes of the device;
respective shape of the available stimulation electrodes of the device;
amperage and/or frequency of driving currents to be applied to individual stimulation electrodes of the device;
electrical ac/dc-driving scheme to be applied to the device;
frequency of driving AC currents;
relative phase and/or time-lag of driving AC currents;
waveform of driving AC currents;
timing-scheme of driving AC currents, in particular time sequence of activation of the stimulation electrodes;

For example, the magnitude/amplitude of the applied AC currents can be a simulation parameter to be varied, because it is possible to achieve the desired current magnitude in the ROI with a superposition of different lower currents delivered from a respective set of electrodes.

In addition, step C) may be performed to maximize a local electrical field strength in at least one region of interest (ROI) extracted from the at least one image in step A), in particular while minimizing the electrical field strength in remaining tissue regions; and/or using a state-of-the-art optimization algorithm, preferably based on a finite-element-method; By such approaches, it can be safeguarded that in remaining regions outside of the at least one region of interest (ROI), extracted from the at least one image in step A), an effective field strength remains below a physiological action potential (AP) of neuronal cells present in these remaining regions ("sub-critical stimulation" c.f. above).

As examples, during step C) at least one, preferably at least two, of the following boundary conditions may be taken into account:
maximum driving voltage and/or driving current available for driving an individual stimulation electrode of the device;
available frequency range for a driving current to be applied to an individual stimulation electrode of the device;
number M of available fixed geometrical arrangements of N stimulation electrodes provided by a limited set of M available different stimulation devices;
maximum number Nₘₐₓ of available stimulation electrodes;
limited number W of different available shapes of stimulation electrodes.

The pre-existing images from which the computer extracts the patient-specific data may be present as a digital data set. For achieving an efficient and meaningful optimization, the at least one pre-existing image may comprise at least one of, preferably at least two of,
a magnetic resonance tomography (MRT) image (3) and/or
a positron emission tomography (PET) image (3) and/or
a X-ray computed tomography (CT) image (3).

A highly useful approach, in particular for efficiently therapy of AD with the device later on, is implemented, if step A) comprises extracting localized concentrations of particular proteins (which define respective regions of interest (ROI)) from pre-existing PET-images. In this case, the PET-images may have been acquired (previously, before performing the method) using protein-specific, in particular radio-active, markers. Such state-of-the-art clinical imaging techniques allow identification of a ROI with high specificity and high spatial resolution, which are both important for the later success of the electrostimulation therapy (which is not part of the method presented herein, however).

The stimulation device can also feature at least one electrode (in particular one of said N stimulation electrodes) configured or (at least) configurable (e.g. electronically) for recording electrical fields generated with some of the (remaining) N stimulation electrodes of the device and/or for recording nerve signals from neuronal tissue, in particular brain waves (which are normally recorded using an electroencephalography (EEG)-device). Such a design has the advantage, for example, that during therapy, the stimulation device can stimulate the brain for a first period of time, followed by recording of brain waves for a second period of time, and so on.

Measurement data obtained with this at least one recording electrode may be used as another valuable input for optimizing the configuration parameters during step B) and C). In other words, electrical fields and or nerve signals, which may have been previously recorded with the stimulation device, can be used as an input to be considered during step B).

For example, an electrical field produced by a subset of the N stimulation electrodes may be read-out with at least one electrode of the device, which is (momentarily or permanently) configured as a recording electrode. This read-out may then serve to rearrange at least one of the N stimulation electrodes (as a possible result of the optimization performed during step B) and C).

This approach may also be done iteratively, such that a loop of stimulation & recording, optimization, followed by repetition of stimulation & recording and subsequent optimization may be achieved. Such a loop can be highly beneficial for achieving optimized electrical stimulation of deep brain regions, in particular taking into account resonance effects (i.e., a synchronization with brain waves, which can be detected by a set of recording electrodes) occurring during stimulation.

For solving the afore-mentioned problem, there is also proposed a method for computing a series of individual sets of optimized configuration parameters. Each of these different sets is computed by performing a method according to one of the claims directed towards a computer-implemented method for computing a patient-specific set of configuration parameters (to be applied to an electrical stimulation device) or as described before.

Each of the individual sets may be intended for configuring the stimulation device prior to a specific therapy session of a series of such sessions. This way, each of the sets can be optimized for enabling a pre-defined electrostimulation-pattern to be generated with the stimulation device during a particular session. Advantageously, the different consecutive electrostimulation-patterns may define a specific electro-therapy scheme that is to be applied to the patient in a series of electro-therapy sessions.

The sets of configuration parameters may differ in at least one of the configuration parameters, for example in an arrangement / location of individual stimulation electrodes and/or in a parameter of an electrical stimulation protocol applied to the stimulation device.

Following this inventive concept, the afore-mentioned problem can also be solved by an electrical stimulation device, as described in the following: The disclosure proposes an electrical stimulation device as introduced at the beginning, which, in addition, is characterized in that the device comprises a number of N non-invasive stimulation electrodes arranged in a 3D-geometry, an electronic driving circuit for providing individual electrical driving voltages or driving currents to the individual stimulation electrodes, and a data interface for configuring said stimulation device with a patient-specific set of configuration parameters.

The device is further distinguished in that the N stimulation electrodes are arranged on a shell of a helmet with patient-specific design that is based on the anatomy of the brain of an individual patient. The shell and hence the helmet are stable in shape. This has the advantage that the relative position of each stimulation electrode with respect to the brain of the patient for whom the helmet is designed is well-known. This is important as the location of the brain with respect to the skull varies from patient to patient, so standard systems with non-patient-specific designs, which only consider the angle or distance of the electrodes w.r.t. the skull, will lead to significant errors in the targeting of the electrostimulation, when used with different patients.

In other words, the disclosure proposes, in particular, to arrange the electrodes of the stimulation device on the shell of the helmet according to positions, which have been determined (in particular optimized) using the computational method described herein. This method can take the anatomy of the brain of an individual patient into account, based on a patient-specific head model, which has been derived in step A) from at least one pre-existing image of the patient's brain. Such a device can deliver a much better accuracy of electrostimulation, in particular when delivering a tACS with multiple focii.

The 3D-geometry in which the stimulation electrodes are arranged is thus patient-specific (i.e., tailored to achieve the focused electrostimulation best-suited for the patient). In other cases, a standard 3D geometry of the electrodes may be used (i.e., the positions of the electrodes may not be changed from patient to patient), but the electrical stimulation protocol derived with the method will then drive these existing electrodes such that a best-case electrostimulation is achieved for the patient. Some of the electrodes of the device may thus not be used at all.

The electrodes of the device can be of a round or angular shape; they can also be designed as a wire or strip. When using strips, for example, the electrodes may be customized to the head of a patient. They can also be embedded in a cloth or mesh of the stimulation device. Such designs are beneficial for efficiently stimulating specific areas of the head, for example.

The 3D-geometry of the stimulation electrodes may be fixed or slightly re-configurable within a certain geometrical adjustment range, depending on the application.

A particular handy design of the stimulation device is thus a shape-stable helmet or bonnet to be worn by a patient on his skull. This helmet/bonnet may carry the N stimulation electrodes, and preferably the driving circuit, and most preferably an electrical power supply for autonomous operation of the driving circuit. The helmet/bonnet may be tailored to the head of a patient, based on pre-existing medical images (MRI/CRT/PET) of the head.

Moreover, for reasons of patient safety, it is preferably if electrical interconnections between the stimulation electrodes and the driving circuit are embedded in the bonnet/helmet, in particular such that they cannot be manipulated by a patient without destructing the bonnet/helmet.

Such a stimulation device may be pre-configured in various ways. For example, a geometrical arrangement of the N stimulation electrodes of the device may be re-configurable, i.e., the electrodes may be movable in the respective 3D-location on the bonnet/helmet and/or they may be changeable in their shape (e.g., by replacing a respective electrode head).

The geometrical arrangement and/or shape of the N stimulation electrodes can also be permanently or initially fixed and hence not be re-configurable.

The (preferably wireless) data interface, on the other hand, may be configured to enable configuration and/or control of the stimulation device via an external, preferably mobile, electronic device, such as a computer or a tablet. That is, the data interface can provide a (preferably wireless and/or bidirectional) data link between the stimulation device and the additional electronic device, such that the stimulation device may be configured with the help of (or even automatically by) the electronic device.

As previously contemplated, the method may also take into account a set of pre-existing different electrical stimulation devices. The afore-mentioned problem is therefore also solved by a set of a limited number M (preferably 2<M<64) of electrical stimulation device, with each device offering a respective number of N individually addressable non-invasive stimulation electrodes. In particular, each of these M stimulation devices may be a stimulation device as described above or as defined in the claims. For solving the problem, the set is characterized in that the stimulation devices comprised in the set differ in terms of their respective number N of available individual stimulation electrodes; and/or in a respective fixed geometrical arrangement of a number of N stimulation electrodes; and/or in a respective individual shape of at least one of the respective stimulation electrodes. Most preferably, the number M may be kept smaller than 10, such that the fabrication effort for providing the set is limited.

In addition, the afore-mentioned problem can be solved by a system for patient-specific electrostimulation of neuronal tissue, the system comprising an electrical stimulation device as described above; and a, preferably mobile, electronic device, which can communicate with and control the electrical stimulation device via a, preferably wireless, data interface of the stimulation device.

The system is further characterized in that the electronic device is configured to transmit a set of configuration parameters to the stimulation device via the data interface of the stimulation device, and these parameters are derived using a method according to the invention.

According to a specific embodiment, the configuration parameters, in particular an electrical stimulation protocol, to be transmitted to the stimulation device prior to a (e.g., daily) therapy session (e.g., of a length of 30 min), may be adapted over time, following a certain electro-therapy scheme. Such adaptions may also be simulated and optimized, preferably prior to a series of therapy sessions to be performed, each time using a computer-implemented method according to the invention, as described previously. This approach allows definition of specific respective sets of defined configuration parameters for each therapy session of a series of therapy sessions.

Alternatively, or additionally, the electronic device may feature an internet interface for enabling remote-control of the electronic device and thereby of the stimulation device. By this approach, an electrostimulation that is later performed with the stimulation device can be live-monitored and/or stopped via a remote-connection to the electronic device, which has been established using its internet interface.

The internet interface may be implemented, for example, using a standard WLAN- or LAN-module built-into the electronic device.

The disclosure also proposes a computer system for patient-specific configuration of an electrical stimulation device, which is designed for electrical stimulation and/or recording of neuronal tissue, in particular of cerebral tissue, for solving the afore-mentioned task. This system may comprise a typical processer, a data storage device coupled to the processor, and optionally a display device. The data storage device stores instructions (in particular in the form of a computer program as specified further below) operable to cause the processor to perform a method according to one of the claims directed towards a method or as described herein.

When the processor operates the instructions, the processor may extract patient-specific data from a pre-existing image (this image may also be present on the data storage device or accessible via a network interface operable by the processor, for example); the processor may then perform a simulation of an electrical field distribution that can be generated with the device when configured with a specific set of configuration parameters (the simulation may start with a pre-defined standard setting of these configuration parameters); this simulation can be performed repeatedly by the processor, each time varying a set of simulation parameters, i.e. in particular varying one of said configuration parameters; and finally the processor may output a final patient-specific set of configuration parameters, which are the result of an optimization performed during iterative repetition of said computer simulation.

The set of configuration parameters now finally optimized for the patient, may then be used to configure and/or choose the stimulation device, which can thus be made ready for performing a patient-specific electrical stimulation with the device (this step of treatment of the patient may thus follow the step of configuring the stimulation device).

There can also be provided a computer program, which solves the afore-mentioned problem. This program, which may preferably be designed as an app to be run on a mobile electronic device such as a tablet or smart phone, comprises instructions, which, when the program is executed by a computer, in particular by said electronic device, cause the computer / said electronic device to carry out a method as defined in the claims 1 to 14.

For achieving a patient-friendly solution, another computer program is proposed, which can also be designed as a mobile app. This second program comprises instructions, which, when carried out by an external mobile electronic device or a computer, cause the computer / electronic device to communicate with an electrical stimulation device (which may be designed / configured as described herein or by the claims 16 to 18) and to configure the stimulation device with a set of configuration parameters that enable patient-specific electrostimulation. Here again, the set of configuration parameters has been derived with a method as defined by the claims 1 to 14.

Alternatively, or additionally, the instructions can also cause the computer / electronic device to establish a remote connection to the electronic device via the internet. This way, the computer program may enable a live monitoring of and/or stopping of an electrostimulation to be performed with the electrical stimulation device.

Such a live monitoring may comprise a monitoring of voltages or driving currents applied to the stimulation electrodes of the electrical stimulation device. In case these voltages or currents exceed maximum values defined to guarantee safe electrostimulation, a monitoring algorithm may automatically stop the performed electrostimulation via the remote-connection and also provide a warning to the user via the electronic device (acoustically and/or optically, e.g., by a banner on the display or the like).

The computer programs mentioned before may be sophisticated further to achieve further benefits for the patient: For example, the program can also comprise instructions, which, if carried out, by the electronic device cause the electronic device to offer the user a cognitive test. This offer may be generated by the electronic device before and/or after an electrostimulation has been performed with the electrical stimulation device. The offer can be presented to the patient in particular using a display and/or loudspeakers; answers form the patient may be recorded using a microphone of the electronic device and/or a touch display.

In addition, the patient can wear the stimulation device while performing such a cognitive test. This has the advantage that recording electrodes of the stimulation device (this may be stimulation electrodes, simply electronically reconfigured as recording electrodes) may be used to record neural signals of the patient during the cognitive test; these signals may be recorded by the electronic device as a bio marker. Hence, the instructions can cause the electronic device to record nerve signals (in particular brain waves) from the patient using a recording electrode of the stimulation device, while the patient performs the test.

This approach allows to digitally document a bio marker, for example a success rate of a cognitive test result, based on a response to that cognitive test, which the user has input into the electronic device via a user interface. In particular, the electronic device may record a time stamp and or parameters of a performed electrostimulation together with the bio marker. This way a highly informative bio marker data set can be formed, which can provide important information to HCP for steering the therapy in the future.

Finally, for solving the afore-mentioned problem, a computer-readable data carrier may be used (in particular for tangibly storing one of said computer programs) comprising instructions, which when executed by a computer, cause the computer to carry out a method according to one of the claims directed towards a method and/or as described herein. The digital output, resulting from carrying out the instructions, will thus be the desired set of configuration parameters, which are optimized for the individual patient and can be used to configure the electrical stimulation device. As a result, the device can provide a patient-specific electrical stimulation that is tailor-made for treating neuronal tissue of a particular patient.

It is obvious, that similar data carriers may be used which comprise instructions defining a computer program as described before or as defined in the claims.

With reference to the accompanying drawings:
- Fig. 1: is a schematic flow diagram, illustrating the individual steps of the method according to the invention,
- Fig. 2: illustrates a system for patient-specific electrostimulation of brain tissue,
- Fig. 3: illustrates patient-specific configuration of an electrical stimulation device, as embedded in a more general concept of providing patient-specific diagnostic, care and therapy, and
- Fig. 4: illustrates another electrical stimulation device in the form of an electrode helmet.

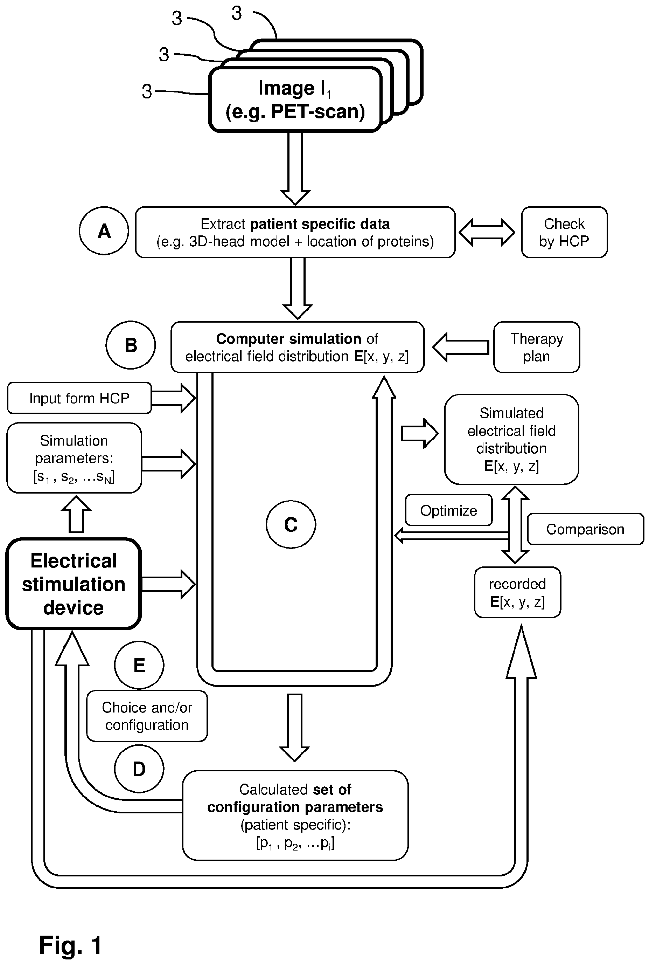

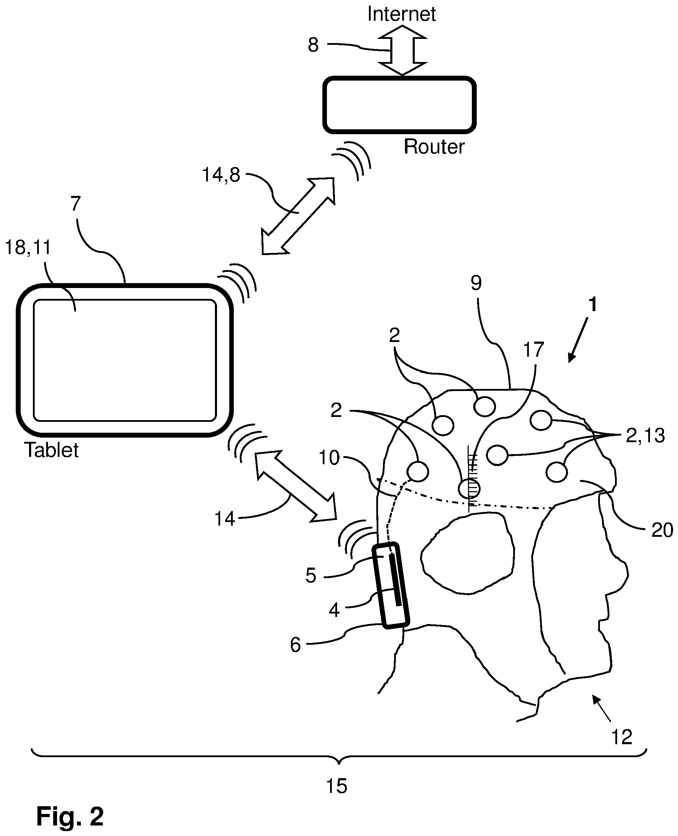

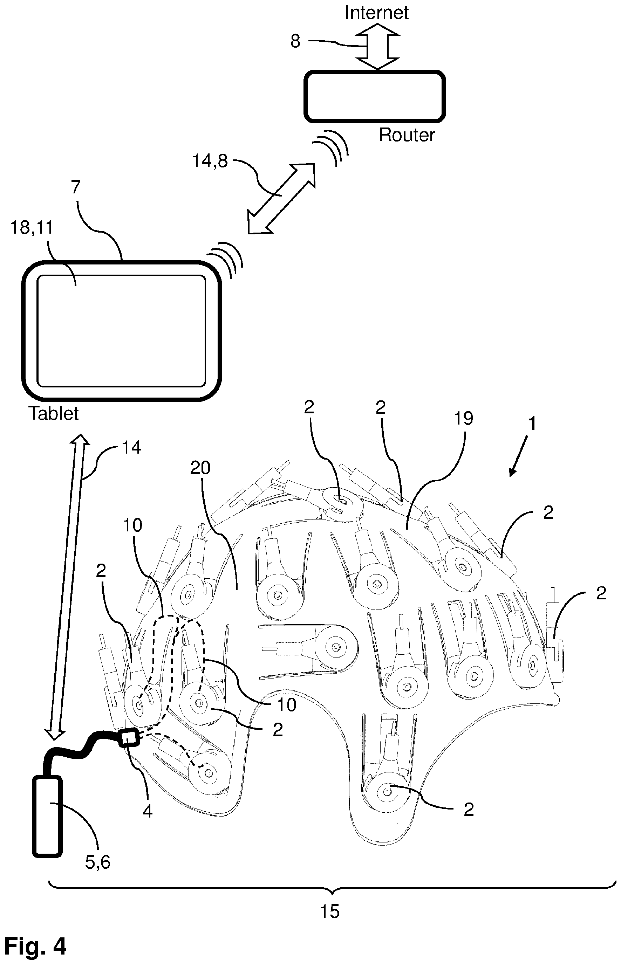

Figure 1 illustrates a computer-implemented method for deriving a patient-specific set of configuration parameters that can be used for configuring and/or choosing an electrical stimulation device 1, as the one shown in Figure 2 oder 4, that is designed for electrical stimulation and/or recording of cerebral tissue.

As previously explained, the method may benefit from pre-existing images 3, e.g. MRT- or PET-scans, obtained from the patients head (as one example) in the hospital, where the necessary imaging equipment is available. The method may then be implemented on any suitable computer system, which may be remote from the hospital, even on another continent. Afterwards, health care professionals (HCP) or the patient himself may be provided with the derived set of optimized configuration parameters and the HCP or the patient himself (in particular remotely assisted via the internet, as will be explained below) may configure the stimulation device 1. After this step, the patient himself may perform an electrostimulation therapy safely himself, in particular at home, using the so-configured device 2.

As shown exemplarily in Figure 1, the method may benefit from a set of pre-existing images 3, which were acquired in a positron emission tomography (PET) scan of the skull of the patient. In a first step A), a processor of a computer system (that executes instructions defining a method as now explained) extracts a number of patient-specific data from the series of images 3, including a 3D-distribution of gray and white matter within the brain of the patient but also localized concentrations of specific proteins, namely amyloid and tau proteins. Based on this data, the processor defines several regions of interests (ROI) to be electro-stimulated.

These regions are presented by the computer system on a display such that a health care professional (HCP) can check and affirm them.

In addition, the processor may process further images 3, for example a series of X-ray computed tomography (CT) or magnetic resonance tomography (MRT) images 3 to extract geometrical data of the skull of the patient, such as distribution of a thickness of the skull bone and of the scalp. From this data, the processor can generate an accurate 3D-model (head model) of the skull that allows precise simulation results.

In a next step B), the computer simulates an electrical field distribution that would be achievable with one particular stimulation device 1, when configured with a specific set of start parameters. These start parameters may define a standard location of a number of N stimulation electrodes, their specific shape but also electrical parameters such as a current strength (amperage), frequency of a certain drive voltage applied to one of the stimulation electrodes (in case of AC-electrostimulation) or relative strengths of currents as distributed among the N electrodes.

Using this start set of simulation parameters, which can also include boundary conditions such as an available maximum voltage or the like, the processor virtually simulates/calculates the electrical field distribution E(x,y,z) that would result in the patient's brain, if the chosen stimulation device 1 would be configured and/or operated with the start parameters (and worn by the patient).

During this simulation, the computer takes into consideration the patient-specific data extracted from the images 3, because the simulation makes use of the 3D-model of the skull calculated from that data. The calculation of the E-field E(x,y,z) is performed by the computer using a state-of-the-art finite element method (FEM); the calculated E-fields E(x,y,z) are also documented in a simulation data file by the computer.

In addition, the computer runs iteratively a series of such simulations, each time varying at least one of said simulation parameters (e.g. virtually changing a certain 3D-location of one of the stimulation electrodes 2, in particular using some random-walk-algorithm) and each time evaluating the calculated electrical field distribution E(x,y,z) using a merit function. In other words, the second step B) is iteratively applied to optimize the generated field distribution E(x,y,z), taking into account the ROI extracted from the pre-existing PET-images 3 with the aim of focusing the simulated field distribution E(x,y,z) in the ROI.

This optimization procedure can be run until no significant progress is made. The resulting set of optimized configuration parameters, which were part of the simulation parameters used in the last simulation, may thus be considered as a "best-possible solution" for a given stimulation device 1.

The whole process may be repeated, starting with a different set of start parameters. For example, a different type of stimulation device 1, featuring a different number of N stimulation electrodes or at least N stimulation electrodes arranged in a different 3d-arrangement, may be chosen by the computer as the starting point of the optimization routine.

The single stimulation devices 1 may also be chosen from a pre-defined set of a limited number of M such devices 1, the devices 1 differing from each other for example in the number N and/or specific 3D-position of the stimulation electrodes 2.

Finally, all "best-case" solutions calculated for each of the M devices 1 may be compared to find the stimulation device 1 and associated optimized set of configuration parameters that best fits the needs of the patient, i.e., which would best enable an efficient electrostimulation therapy using one particular stimulation device 1. Of course, such a simulation can also be performed for only one specific type of stimulation device 1.

As shown in Figure 1, the output of the computer-implemented method may thus be the information about which one of the M stimulation devices 1 of the set is best suited and/or which are the best-suited configuration parameters for a particular stimulation device 1, given the skull and brain structure of the patient as described by the images 3.

Based on this output, one of the M stimulation devices 1 (e.g., a bonnet 9 or helmet 19 with a size and electrode configuration best suited for the patient) may be chosen (step E) and this particular device 1 may then be configured (step D) using the optimized set of configuration parameters.

As illustrated in Figure 1, a therapy plan, defining a specific electro-therapy scheme to be applied to the patient over a series of electro-therapy sessions (e.g., one session / day, with varying electro-stimulation), may be considered. In fact, the method just explained can be performed repeatedly to calculate a series of individual sets of optimized configuration parameters. Each set can then be used prior to a specific therapy session of the series to configure the stimulation device 1. By applying the method, each set can be optimized (prior to the therapy) for enabling a pre-defined electrostimulation-pattern to be generated with the stimulation device 1 during one of the sessions.

Illustrated in Figure 1 is also that a health care professional (HCP) may provide further input for the simulation to be performed in step B. For example, the HCP may initially choose the stimulation device 1 (e.g., based on medical judgement or primary clinical trials) for which the configuration parameters are to be optimized. The HCP may deliver such information to the computer system by choosing a certain set of start simulation parameters [s₁, s₂, ... s_{N}]. Moreover, HCPs can also deliver E-field distributions E(x,y,z), for example as recorded with a particular stimulation device 1 configured with a specific set of configuration parameters [p₁ , p₂, ... pᵢ]. Such data can then be compared (by the HCP or the computer itself) with the simulated field distribution and employed for further optimization of the configuration parameters.

Figure 2 schematically sketches a system 15 for patient-specific electrostimulation according to the invention, comprising an electrical stimulation device 1 and a mobile electronic device 7, namely a tablet. The stimulation device 1 is designed as a bonnet 9 featuring a shell 20 (located above the dot-dashed line in Figure 2 and below the bonnet 9), which can be worn by the patient on his head 12. The bonnet 9, or to be more precise the shell 20, carries a number of N non-invasive stimulation electrodes 2 arranged in a 3D-geometry, an electronic driving and read-out circuit 4 that can provide individual electrical driving voltages to the individual electrodes 2 according to an electrical stimulation protocol, and an electrical power supply 6 for autonomous operation of the circuit 4. As the shell 20 is stable in shape and features a patient-specific shape (matching the skull of the patient), the position of each electrode 2 relative to the patient's brain is known as soon as the patient puts on the bonnet 9.

As illustrated, some of the electrodes 2 can also be configured electronically as electrical recording electrodes 13 than can be read-out with the electronic circuit 4. Thereby, nerve signals but also E-fields generated with the remaining stimulation electrodes 2 can be recorded with the device 1, when it is worn on the head by the patient.

The device 1 also features a wireless data interface 5 for communication with the tablet 7 via a bidirectional wireless connection 14. The tablet 7 itself is connected to and accessible from the internet via an internet interface 8 that is realized by wirelessly establishing a second connection 14 between the tablet 7 and an internet router.

The data interface 5 of the device 1 is primarily designed to configure the device 1 with a set of optimized configuration parameters derived with the method according to Figure 1. The data interface 5 may be used, however, also for delivering digital data documenting a performed electrostimulation with the device or recorded E-fields and/or nerve signals to the tablet 7. In other words, the data interface 5 of the device 1 is configured to enable configuration and/or control of the stimulation device 1 via the tablet or any other suitable electronic device 7.

Part of the configuration parameters, for examples "best-suited" locations of the individual stimulation electrodes 2 within a certain adjustment range 17, may also be presented to the patient on the display 18 of the tablet 7, such that the patient or a HCP can configure the device 1 accordingly.

One advantage of the approach illustrated in Figure 2 is that is allows remote-control and live-supervision of an electrostimulation therapy performed with the device 1: Through the internet interface 8, a remote-connection to the electronic device 7 can be established and from there to the device 1, in particular for delivering the configuration parameters from a remote computer system or database to the device 1. This remote-connection can also enable an HCP or a computer system to live-monitor, supervise and - in case of danger - to stop an electrostimulation performed with the stimulation device 1.

Another advantage discussed before is that such a system 15 enables tracking of the success of an electrostimulation therapy performed with the device 1 over time, based on input from cognitive tests that the patient has performed using the tablet 7. Via the tablet 7, this input/feedback may be easily delivered to a remote computer system, e.g., of a hospital.

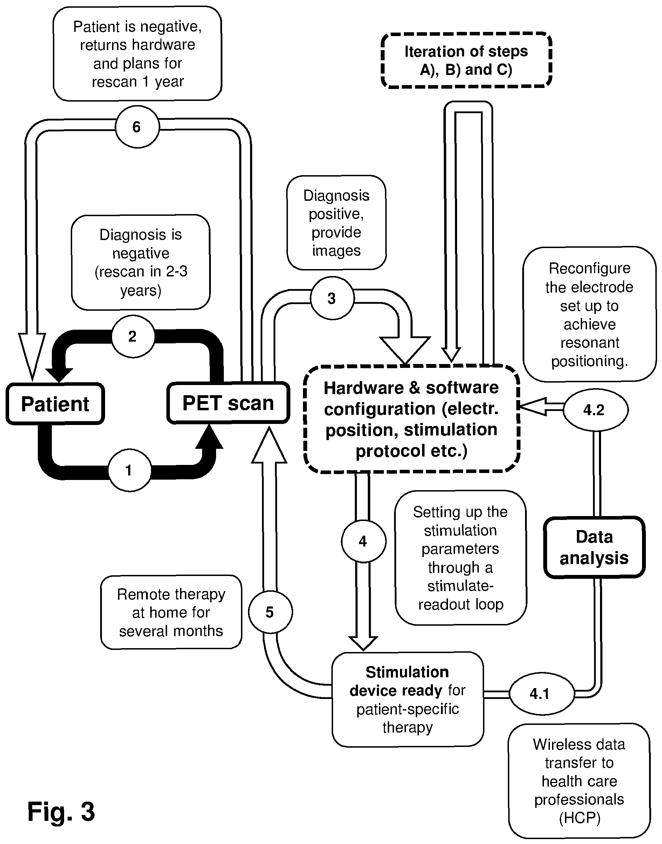

Figure 3 finally illustrates, that the calculation of a set of optimized configuration parameters, and the consequent configuration of a chosen electrical stimulation device 1 may form an important piece of a more general concept of providing patient-specific diagnostic, care and electrostimulation therapy: The hardware and software configuration that is achievable with the methods and devices presented herein, including computer-implemented iterations of the method steps A)-C) for improving this configuration / setup, can form the basis of a complex cycle of regular diagnostic scans (using MRT, PET and the like), experimental data from cognitive tests performed by the patient, data analysis and patient-specific treatment. The method and devices presented herein provide the patient and the HCP with a best-possible stimulation solution for a given design and adaptability of a particular device 1. Figure 4 presents another implementation of a stimulation device 1 designed for multi-focal tACS. The device 1 is designed as a helmet 19 with a shell 20, that has been fabricated by additive manufacturing in a patient-specific shape. The shell 20 is therefore stable in shape and fits neatly to the skull of the patient, for whom it has been designed. The stimulation electrodes 2 are arranged on the shell 20 according to positions, which have been computed (as part of the configuration parameters) using the method according to the invention. When the patient is wearing this helmet 19 and the electrodes 2 are driven according to the electrical stimulation protocol optimized with the method, it is possible to electro-stimulate several ROI inside the brain of the patient with high spatial accuracy.

In summary, a computer-implemented method is proposed that allows patient-specific choice and/or configuration of an electrical stimulation device 1 that is designed and usable for electrostimulation therapy of neuronal tissue, in particular in the brain. The method benefits from pre-existing images 3 acquired using a medical imaging technique such as MRT or PET. Based on data extracted from such an image 3, a computer simulates possible electrical field distributions E(x,y,z) which would be achievable if the device 1 would be configured with a certain set of configuration parameters. By varying these parameters and repeating the simulations, the method allows optimization of the configuration parameters for a given design of the device 1. As a result, the method delivers a set of optimized configuration parameters, which, if applied to the device 1, allow a "best-case" electrostimulation, tailor-made for the need of the patient, with the device (cf. Figure 1).

### List of reference numerals

- 1: electrical stimulation device
- 2: stimulation electrode (of 1)
- 3: images (e.g., acquired by MRI, CT, PET or like methods)
- 4: electronic driving circuit (of 1)
- 5: data interface (of 1)
- 6: power supply (of 1)
- 7: electronic device
- 8: internet interface (of 7)
- 9: bonnet
- 10: interconnections (between 2 and 4)
- 11: user interface (e.g., touch display, microphone, external keyboard / mouse etc.)
- 12: patient's head
- 13: recording electrode
- 14: wireless connection (bidirectional)
- 15: system for patient-specific electrostimulation
- 16: computer system
- 17: adjustment range (of position of 2)
- 18: display
- 19: helmet
- 20: shell

## Claims

1. **Computer-implemented method** for computing a patient-specific set of configuration parameters suitable for configuring and/or choosing an electrical stimulation device (1) designed for electrical stimulation and/or recording of neuronal tissue, in particular of cerebral tissue, the method comprising the following steps:
A) extracting patient-specific data including at least one region of interest (ROI) from at least one pre-existing image (3) previously acquired using a medical imaging technique;
B) computer simulation of an electrical field distribution that can be generated using the electrical stimulation device (1) when configured with a specific set of configuration parameters defining a specific transcranial alternating current stimulation (tACS) scheme, taking into consideration the extracted patient-specific data;
C) Variation of a set of simulation parameters during iterative application of step B) to compute the patient-specific set of configuration parameters, which are thus optimized for the patient;
D) configuring the stimulation device (1) with the derived set of configuration parameters such that the stimulation device (1) is configured to focus the electrostimulation onto the at least one region of interest (ROI);

2. Method according to claim 1, wherein the at least one region of interest (ROI) is extracted in step A) exclusively from pre-existing positron emission tomography (PET) images (3),
- preferably based on localized concentrations of specific proteins, preferably amyloid and/or tau proteins, visible in the PET-images (3),
- preferably wherein a head model as part of the patient-specific data is extracted in step A) exclusively from the PET-images (3).

3. Method according to claim 1 or 2,
- wherein step C) comprises a variation of a physical configuration of N stimulation electrodes (2) of the stimulation device (1),
- preferably wherein the variation comprises a change of a location of one of the N stimulation electrodes (2) or a size and/or shape of one of the N stimulation electrodes (2), and/or
- wherein N stimulation electrodes (2) of the stimulation device (1) can generate said electrical field distribution.

4. Method according to any of the previous claims, wherein the configuration parameters comprise at least one, preferably at least two, of the following parameters:
- a specific geometrical arrangement of N stimulation electrodes (2) of the stimulation device (1) achievable by re-arranging the electrodes (2) within a limited geometrical adjustment range offered by the stimulation device (1);
- a specific geometrical arrangement of N stimulation electrodes (2) belonging to one particular stimulation device (1) comprised in a limited number M of stimulation devices (1) each featuring a pre-defined, preferably non-reconfigurable, respective arrangement of the N stimulation electrodes (2);
- a relative distribution of N driving currents or driving voltages to be applied to N stimulation electrodes (2) of the stimulation device (1);
- a relative distribution of phase lags of N driving AC currents or N driving AC voltages to be applied to N stimulation electrodes (2) of the stimulation device (1);
- a timing-scheme for N driving AC voltages to be applied to N stimulation electrodes (2) of the stimulation device (1), preferably including N specific relative time-lags and/or a respective time sequence of activation of each of the stimulation electrodes;
- a set of N waveforms of N driving AC voltages to be applied to N stimulation electrodes (2) of the stimulation device (1).

5. Method according to any of the previous claims, wherein the configuration parameters comprise a patient-specific electrical stimulation protocol for driving a number of N stimulation electrodes (2) of the device (1) in a transcranial alternating current stimulation (tACS) scheme,
- preferably wherein the stimulation protocol comprises at least one, preferably at least two, of the following electrical stimulation parameters:
- amperage, preferably AC magnitude, and/or
- frequency and/or
- ac driving scheme and/or
- relative phase and/or
- waveform and/or
- time-lag
of individual driving currents to be applied to the individual stimulation electrodes (2) of the stimulation device (1).

6. Method according to claim 5, wherein step
D) comprises configuring the stimulation device (1) with said electrical stimulation protocol.

7. Method according to any of the previous claims, wherein the method further comprises a step of
E) choosing said electrical stimulation device (1) out of a pre-defined set of M different stimulation devices (1) based on the computed set of configuration parameters,
- preferably wherein the M stimulation devices (1) comprised in the pre-defined set differ:
- in their respective number N of available individual stimulation electrodes (2) and/or
- in a respective, fixed or adjustable, geometrical arrangement of a number of N stimulation electrodes (2) and/or
- in a respective individual shape of at least one of the respective stimulation electrodes (2).

8. Method according to any of the previous claims, wherein the patient-specific data extracted in step A) comprise at least one, preferably ate least two, of the following patient-specific parameters:
- geometrical data of a skull of the patient, such as distribution of a thickness of the skull bone and/or of the scalp;
- 3D-distribution of brain tissue, preferably size of the patient's brain and/or distribution of gray and white matter within the brain of the patient;
- localized concentrations of specific proteins, in particular of amyloid and/or tau proteins, within the brain of the patient;
- localized occurrence of atrophy in the brain of the patient;
- 3d-coordinates of at least one region of interest (ROI) to be electro-stimulated with the device (1);
- preferably wherein the optimization performed in step B) takes into account at least one of the patient-specific parameters for computing said set of configuration parameters, and/or
- wherein the optimization performed in step B) is based on a geometrical head model derived from the patient-specific data extracted in step A).

9. Method according to any of the previous claims, wherein the simulation parameters varied in step C) comprise at least one, preferably at least two, of the following parameters:
- number N of available stimulation electrodes (2) of the device (1);
- geometrical arrangement of N available stimulation electrodes (2) of the device (1);
- respective shape of the available stimulation electrodes (2) of the device (1);
- amperage and/or frequency of driving currents to be applied to individual stimulation electrodes (2) of the device (1);
- electrical ac/dc-driving scheme to be applied to the device (1);

10. Method according to any of the previous claims, wherein step C) is performed
- to maximize a local electrical field strength in at least one region of interest (ROI) extracted from the at least one image in step A), preferably while minimizing the electrical field strength in remaining tissue regions and/or
- using an optimization algorithm, preferably based on a finite-element-method, and/or
- such that in remaining regions outside of the at least one region of interest (ROI), extracted from the at least one image in step A), an effective field strength remains below a physiological action potential (AP) of neuronal cells present in these remaining regions.

11. Method according to any of the previous claims, wherein during step C) at least one, preferably at least two, of the following boundary conditions are taken into account:
- maximum driving voltage and/or driving current available for driving an individual stimulation electrode (2) of the device (1);
- available frequency range for a driving current to be applied to an individual stimulation electrode (2) of the device (1);
- number M of available fixed geometrical arrangements of N stimulation electrodes (2) provided by a limited set of M available different stimulation devices (1);
- maximum number Nₘₐₓ of available stimulation electrodes (2) ;
- limited number W of different available shapes of stimulation electrodes (2).

12. Method according to any of the previous claims, wherein the at least one pre-existing image (3) comprises at least one of, preferably at least two of,
- a magnetic resonance tomography (MRT) image (3) and/or
- a positron emission tomography (PET) image (3) and/or
- a X-ray computed tomography (CT) image (3) and/or
- wherein step A) comprises extracting localized concentrations of particular proteins, defining respective regions of interest (ROI), from pre-existing PET-images (3), preferably wherein step A) comprises PET-images (3) acquired using protein-specific, preferably radio-active, markers.

13. Method according to any of the previous claims, wherein the stimulation device (1) features at least one electrode, preferably one of the N stimulation electrodes (2), configured or configurable for recording
- electrical fields generated with some of the N stimulation electrodes (2) and/or
- nerve signals from neuronal tissue, preferably brain waves,
- preferably wherein, electrical fields and or nerve signals previously recorded with the stimulation device (1) are used as an input for optimizing the configuration parameters during step B) and C).

14. Method according to any of the previous claims, wherein the stimulation device (1) features
- a number of N non-invasive stimulation electrodes (2) arranged in a 3D-geometry,
- an electronic driving circuit (4) for providing individual electrical driving voltages or driving currents to the individual stimulation electrodes (2),
- a data interface (5) for configuring said stimulation device (1) with a patient-specific set of configuration parameters,
- wherein the N stimulation electrodes (2) are arranged on a stable-shape shell (20) of a helmet (19) or bonnet (9) with patient-specific design that is based on the anatomy of the brain of an individual patient and which is stable in shape,
- and wherein the stimulation device (1) is configured with a set of configuration parameters that have been derived with a method according to one of the claims 1 to 13,
- in particular wherein the geometrical arrangement of the N stimulation electrodes (2) is re-configured.

15. **Method for computing a series of individual sets of optimized configuration parameters,**
- wherein each set is computed by performing a method according to any of the preceding claims 1 to 14,
- preferably wherein each of the individual sets is intended for configuring the stimulation device (1) prior to a specific therapy session of a series of such sessions, and/or
- wherein each of the sets is optimized for enabling a pre-defined electrostimulation-pattern to be generated with the stimulation device (1),
- preferably wherein the different consecutive electrostimulation-patterns define a specific electro-therapy scheme.

16. **Computer program,** preferably designed as an app to be run on a mobile electronic device (7), comprising instructions, which, when the program is executed by a computer/said electronic device (7), cause the computer/said electronic device (7) to carry out a method according to one of the claims 1-14.

## Patentansprüche

1. Computerimplementiertes Verfahren zum Berechnen eines patientenspezifischen Satzes von Konfigurationsparametern, der zum Konfigurieren und/oder Wählen einer Vorrichtung zur elektrischen Stimulation (1) geeignet ist, die zur elektrischen Stimulation und/oder Aufzeichnung von Nervengewebe, insbesondere von Hirngewebe, konzipiert ist, wobei das Verfahren die folgenden Schritte umfasst:
A) Extrahieren von patientenspezifischen Daten, die mindestens einen Bereich von Interesse (region of interest - ROI) beinhalten, aus mindestens einer zuvor bestehenden Aufnahme (3), die zuvor unter Verwendung einer medizinischen bildgebenden Technik erfasst worden ist;
B) Computersimulation einer elektrischen Feldverteilung, die unter Verwendung der elektrischen Stimulationsvorrichtung (1) erzeugt werden kann, wenn sie mit einem spezifischen Satz von Konfigurationsparametern konfiguriert ist, die ein spezifisches tACS(transkranielle Wechselstromstimulation
- transcranial alternating current stimulation)-Schema unter Berücksichtigung der extrahierten patientenspezifischen Daten definieren;
C) Variation eines Satzes von Simulationsparametern während einer iterativen Anwendung von Schritt B) zum Berechnen des patientenspezifischen Satzes von Konfigurationsparametern, die somit für den Patienten optimiert werden;
D) Konfigurieren der Stimulationsvorrichtung (1) mit dem abgeleiteten Satz von Konfigurationsparametern, sodass die Stimulationsvorrichtung (1) dazu konfiguriert ist, die Elektrostimulation auf den mindestens einen Bereich von Interesse (ROI) zu fokussieren;

2. Verfahren nach Anspruch 1, wobei der mindestens eine Bereich von Interesse (ROI) in Schritt A) ausschließlich aus zuvor bestehenden PET(Positronen-Emissions-Tomographie - positron emission tomography)-Aufnahmen (3) extrahiert wird,
- bevorzugt basierend auf lokalisierten Konzentrationen spezifischer Proteine, bevorzugt Amyloid- und/oder Tau-Proteinen, die in den PET-Aufnahmen (3) sichtbar sind,
- wobei bevorzugt in Schritt A) ein Kopfmodell als Teil der patientenspezifischen Daten ausschließlich aus den PET-Aufnahmen (3) extrahiert wird.

3. Verfahren nach Anspruch 1 oder 2,
- wobei Schritt C) eine Variation einer physischen Konfiguration aus N Stimulationselektroden (2) der Stimulationsvorrichtung (1) umfasst,
- wobei bevorzugt die Variation eine Änderung einer Lage einer der N Stimulationselektroden (2) oder einer Größe und/oder Form einer der N Stimulationselektroden (2) umfasst und/oder
- wobei N Stimulationselektroden (2) der Stimulationsvorrichtung (1) die elektrische Feldverteilung erzeugen können.

4. Verfahren nach einem der vorhergehenden Ansprüche, wobei die Konfigurationsparameter mindestens einen, bevorzugt mindestens zwei, der folgenden Parameter umfassen:
- eine spezifische geometrische Anordnung von N Stimulationselektroden (2) der Stimulationsvorrichtung (1), die durch Neuanordnen der Elektroden (2) innerhalb eines begrenzten geometrischen Anpassungsbereichs, der durch die Stimulationsvorrichtung (1) angeboten wird, erreichbar ist;
- eine spezifische geometrische Anordnung von N Stimulationselektroden (2), die zu einer spezifischen Stimulationsvorrichtung (1) gehören, die in einer begrenzten Anzahl M von Stimulationsvorrichtungen (1) enthalten ist, die jeweils eine vordefinierte, bevorzugt nicht rekonfigurierbare, jeweilige Anordnung der N Stimulationselektroden (2) aufweist;
- eine relative Verteilung von N Ansteuerströmen oder Ansteuerspannungen, die auf N Stimulationselektroden (2) der Stimulationsvorrichtung (1) aufgebracht werden sollen;
- eine relative Verteilung von Phasenverzögerungen von N Ansteuer-Wechselströmen oder N Ansteuer-Wechselspannungen, die auf N Stimulationselektroden (2) der Stimulationsvorrichtung (1) aufgebracht werden sollen;
- ein Zeitsteuerungsschema für N Ansteuer-Wechselspannungen, die auf N Stimulationselektroden (2) der Stimulationsvorrichtung (1) aufgebracht werden sollen, das bevorzugt N spezifische relative Zeitverzögerungen und/oder eine jeweilige Zeitsequenz der Aktivierung von jeder der Stimulationselektroden beinhaltet;
- einen Satz von N Wellenformen von N Ansteuer-Wechselspannungen, die auf N Stimulationselektroden (2) der Stimulationsvorrichtung (1) aufgebracht werden sollen.

5. Verfahren nach einem der vorhergehenden Ansprüche, wobei die Konfigurationsparameter ein patientenspezifisches elektrisches Stimulationsprotokoll zum Ansteuern einer Anzahl von N Stimulationselektroden (2) der Vorrichtung (1) in einem tACS(transkranielle Wechselstromstimulation - transcranial alternating current stimulation)-Schema umfassen,
- wobei das Stimulationsprotokoll bevorzugt mindestens einen, bevorzugt mindestens zwei, der folgenden elektrischen Stimulationsparameter umfasst:
- Stromstärke, bevorzugt Wechselstromstärke, und/oder
- Frequenz und/oder
- Wechselstrom-Ansteuerschema und/oder
- relative Phase und/oder
- Wellenform und/oder
- Zeitverzögerung
individueller Ansteuerströme, die auf die individuellen Stimulationselektroden (2) der Stimulationsvorrichtung (1) aufgebracht werden sollen.

6. Verfahren nach Anspruch 5, wobei Schritt D) das Konfigurieren der Stimulationsvorrichtung (1) mit dem elektrischen Stimulationsprotokoll umfasst.

7. Verfahren nach einem der vorhergehenden Ansprüche, wobei das Verfahren ferner einen Schritt E) Wählen der elektrischen Stimulationsvorrichtung (1) aus einem vordefinierten Satz von M unterschiedlichen Stimulationsvorrichtungen (1) basierend auf dem berechneten Satz von Konfigurationsparametern umfasst,
- wobei sich die M Stimulationsvorrichtungen (1), die in dem vordefinierten Satz enthalten sind, bevorzugt wie folgt unterscheiden:
- in ihrer jeweiligen Anzahl N verfügbarer individueller Stimulationselektroden (2) und/oder
- in einer jeweiligen festen oder anpassbaren geometrischen Anordnung einer Anzahl von N Stimulationselektroden (2) und/oder
- in einer jeweiligen individuellen Form von mindestens einer der jeweiligen Stimulationselektroden (2).

8. Verfahren nach einem der vorhergehenden Ansprüche, wobei die in Schritt A) extrahierten patientenspezifischen Daten mindestens einen, bevorzugt mindestens zwei, der folgenden patientenspezifischen Parameter umfassen:
- geometrische Daten des Schädels eines Patienten, wie etwa Verteilung einer Dicke des Schädelknochens und/oder der Kopfhaut;
- 3D-Verteilung des Hirngewebes, bevorzugt Größe des Hirns des Patienten und/oder Verteilung der grauen und der weißen Substanz im Hirn des Patienten;
- lokalisierte Konzentrationen spezifischer Proteine, insbesondere von Amyloid- und/oder Tau-Proteinen, im Hirn des Patienten;
- lokalisiertes Auftreten einer Atrophie im Hirn des Patienten;
- 3D-Koordinaten von mindestens einem Bereich von Interesse (ROI), der mit der Vorrichtung (1) elektrostimuliert werden soll;
- wobei bevorzugt die in Schritt B) durchgeführte Optimierung mindestens einen der patientenspezifischen Parameter zum Berechnen des Satzes von Konfigurationsparametern berücksichtigt und/oder
- wobei die in Schritt B) durchgeführte Optimierung auf einem geometrischen Kopfmodell basiert, das von den in Schritt A) extrahierten patientenspezifischen Daten abgeleitet ist.

9. Verfahren nach einem der vorhergehenden Ansprüche, wobei die in Schritt C) variierten Simulationsparameter mindestens einen, bevorzugt mindestens zwei, der folgenden Parameter umfassen:
- Anzahl N verfügbarer Stimulationselektroden (2) der Vorrichtung (1);
- geometrische Anordnung von N verfügbaren Stimulationselektroden (2) der Vorrichtung (1);
- jeweilige Form der verfügbaren Stimulationselektroden (2) der Vorrichtung (1);
- Stromstärke und/oder Frequenz der Ansteuerströme, die auf individuelle Stimulationselektroden (2) der Vorrichtung (1) aufgebracht werden sollen;
- elektrisches Wechselstrom-/Gleichstrom-Ansteuerschema, das auf die Vorrichtung (1) aufgebracht werden soll;

10. Verfahren nach einem der vorhergehenden Ansprüche, wobei Schritt C) wie folgt durchgeführt wird,
- um eine lokale elektrische Feldstärke in mindestens einem Bereich von Interesse (ROI), der aus der mindestens einen Aufnahme in Schritt A) extrahiert wurde, zu maximieren, bevorzugt während die elektrische Feldstärke in verbleibenden Gewebebereichen minimiert wird, und/oder
- durch Verwenden eines Optimierungsalgorithmus, bevorzugt basierend auf einem Finite-Elemente-Verfahren, und/oder
- derart, dass in den verbleibenden Bereichen außerhalb des mindestens einen Bereichs von Interesse (ROI), der aus der mindestens einen Aufnahme in Schritt A) extrahiert wurde, eine effektive Feldstärke unterhalb eines physiologischen Aktionspotenzials (AP) von Nervenzellen bleibt, die in diesen verbleibenden Bereichen vorhanden sind.

11. Verfahren nach einem der vorhergehenden Ansprüche, wobei während Schritt C) mindestens eine, bevorzugt mindestens zwei, der folgenden Rahmenbedingungen berücksichtigt werden:
- maximale/r Ansteuerspannung und/oder Ansteuerstrom, die zum Ansteuern einer individuellen Stimulationselektrode (2) der Vorrichtung (1) verfügbar sind;
- verfügbarer Frequenzbereich für einen Ansteuerstrom, der auf eine individuelle Stimulationselektrode (2) der Vorrichtung (1) aufgebracht werden soll;
- Anzahl M verfügbarer fester geometrischer Anordnungen von N Stimulationselektroden (2), die durch einen begrenzten Satz von M verfügbaren unterschiedlichen Stimulationsvorrichtungen (1) bereitgestellt werden;
- maximale Anzahl Nₘₐₓ verfügbarer Stimulationselektroden (2) ;
- begrenzte Anzahl W unterschiedlicher verfügbarer Formen von Stimulationselektroden (2).

12. Verfahren nach einem der vorhergehenden Ansprüche, wobei die mindestens eine zuvor bestehende Aufnahme (3) mindestens eines, bevorzugt mindestens zwei, von Folgenden umfasst:
- eine MRT(magnetische Resonanztomographie - magnetic resonance tomography)-Aufnahme (3) und/oder
- eine PET(Positronen-Emissions-Tomographie - positron emission tomography)-Aufnahme (3) und/oder
- eine CT(Röntgen-Computertomographie - X-ray computed tomography)-Aufnahme (3) und/oder
- wobei Schritt A) das Extrahieren lokalisierter Konzentrationen spezifischer Proteine, die jeweilige Bereiche von Interesse (ROI) definieren, aus zuvor bestehenden PET-Aufnahmen (3) umfasst, wobei bevorzugt Schritt A) PET-Aufnahmen (3) umfasst, die unter Verwendung proteinspezifischer, bevorzugt radioaktiver, Marker erfasst wurden.

13. Verfahren nach einem der vorhergehenden Ansprüche, wobei die Stimulationsvorrichtung (1) mindestens eine Elektrode, bevorzugt eine der N Stimulationselektroden (2), aufweist, die zur Aufzeichnung von Folgenden konfiguriert oder konfigurierbar sind:
- elektrischen Feldern, die mit einigen der N Stimulationselektroden (2) erzeugt wurden, und/oder
- Nervensignalen von Nervengewebe, bevorzugt Hirnwellen,
- wobei bevorzugt elektrische Felder und/oder Nervensignale, die zuvor mit der Stimulationsvorrichtung (1) aufgezeichnet wurden, als eine Eingabe zum Optimieren der Konfigurationsparameter während Schritt B) und C) verwendet werden.

14. Verfahren nach einem der vorhergehenden Ansprüche, wobei die Stimulationsvorrichtung (1) folgendes aufweist:
- eine Anzahl von N nicht-invasiven Stimulationselektroden (2), die in einer 3D-Geometrie angeordnet sind,
- eine elektronische Ansteuerschaltung (4) zum Bereitstellen von individuellen elektrischen Ansteuerspannungen oder Ansteuerströmen an die individuellen Stimulationselektroden (2),
- eine Datenschnittstelle (5) zum Konfigurieren der Stimulationsvorrichtung (1) mit einem patientenspezifischen Satz von Konfigurationsparametern,
- wobei die N Stimulationselektroden (2) an einer Schale von stabiler Form (20) eines Helms (19) oder einer Haube (9) mit einer patientenspezifischen Gestaltung angeordnet sind, die auf der Anatomie des Hirns eines individuellen Patienten basiert und die in der Form stabil ist,
- und wobei die Stimulationsvorrichtung (1) mit einem Satz von Konfigurationsparametern konfiguriert ist, die mit einem Verfahren gemäß einem der Ansprüche 1 bis 13 abgeleitet wurden,
- wobei insbesondere die geometrische Anordnung der N Stimulationselektroden (2) neu konfiguriert wird.

15. Verfahren zum Berechnen einr Reihe individueller Sätze optimierter Konfigurationsparameter,
- wobei jeder Satz berechnet wird, indem ein Verfahren nach einem der vorhergehenden Ansprüche 1 bis 14 durchgeführt wird,
- wobei bevorzugt jeder der individuellen Sätze zum Konfigurieren der Stimulationsvorrichtung (1) vor einer spezifischen Therapiesitzung einer Reihe derartiger Sitzungen vorgesehen ist, und/oder
- wobei jeder der Sätze zum Aktivieren eines vordefinierten Elektrostimulationsmusters, das mit der Stimulationsvorrichtung (1) erzeugt werden soll, optimiert ist,
- wobei bevorzugt die unterschiedlichen aufeinanderfolgenden Elektrostimulationsmuster ein spezifisches Elektrotherapieschema definieren.

16. Computerprogramm, bevorzugt als eine App konzipiert, die auf einer mobilen elektronischen Vorrichtung (7) ausgeführt werden soll, umfassend Anweisungen, die, wenn das Programm durch einen Computer/die elektronische Vorrichtung (7) ausgeführt wird, bewirken, dass der Computer/die elektronische Vorrichtung (7) ein Verfahren nach einem der Ansprüche 1-14 durchführt.

## Revendications

1. Procédé mis en oeuvre par ordinateur pour calculer un ensemble spécifique à un patient de paramètres de configuration appropriés pour configurer et/ou choisir un dispositif de stimulation électrique (1) conçu pour la stimulation électrique et/ou l'enregistrement de tissu neuronal, en particulier de tissu cérébral, le procédé comprenant les étapes suivantes :
A) extraire des données spécifiques à un patient incluant au moins une région d'intérêt (ROI) à partir d'au moins une image préexistante (3) précédemment acquise en utilisant une technique d'imagerie médicale ;
B) une simulation informatique d'une distribution de champ électrique qui peut être générée à en utilisant le dispositif de stimulation électrique (1) lorsqu'il est configuré avec un ensemble spécifique de paramètres de configuration définissant un schéma de stimulation transcrânienne à courant alternatif (tACS) spécifique, en tenant compte des données spécifiques à un patient extraites ;
C) une variation d'un ensemble de paramètres de simulation au cours de l'application itérative de l'étape B) pour calculer l'ensemble spécifique à un patient de paramètres de configuration, qui sont ainsi optimisés pour le patient ;
D) configurer le dispositif de stimulation (1) avec l'ensemble dérivé de paramètres de configuration de sorte que le dispositif de stimulation (1) soit configuré pour focaliser l'électrostimulation sur la au moins une région d'intérêt (ROI) ;

2. Procédé selon la revendication 1, dans lequel la au moins une région d'intérêt (ROI) est extraite à l'étape A) exclusivement à partir d'images de tomographie par émission de positons (TEP) préexistantes (3),
- de préférence sur la base de concentrations localisées de protéines spécifiques, de préférence des protéines amyloïdes et/ou tau, visibles dans les images de TEP (3),
- de préférence dans lequel un modèle de tête faisant partie des données spécifiques à un patient est extrait à l'étape A) exclusivement à partir des images de TEP (3).

3. Procédé selon la revendication 1 ou la 2,
- dans lequel l'étape C) comprend une variation d'une configuration physique de N électrodes de stimulation (2) du dispositif de stimulation (1),
- de préférence dans lequel la variation comprend un changement d'un emplacement de l'une des N électrodes de stimulation (2) ou de la taille et/ou de la forme de l'une des N électrodes de stimulation (2), et/ou
- dans lequel N électrodes de stimulation (2) du dispositif de stimulation (1) peuvent générer ladite distribution de champ électrique.

4. Procédé selon l'une quelconque des revendications précédentes, dans lequel les paramètres de configuration comprennent au moins un, de préférence au moins deux, des paramètres suivants :
- un agencement géométrique spécifique de N électrodes de stimulation (2) du dispositif de stimulation (1) réalisable par réagencement des électrodes (2) dans une plage de réglage géométrique limitée offerte par le dispositif de stimulation (1) ;
- un agencement géométrique spécifique de N électrodes de stimulation (2) appartenant à un dispositif de stimulation particulier (1) compris dans un nombre limité M de dispositifs de stimulation (1) caractérisés chacun par un agencement respectif prédéfini, de préférence non reconfigurable, des N électrodes de stimulation (2) ;
- une distribution relative de N courants d'entraînement ou tensions d'entraînement à appliquer à N électrodes de stimulation (2) du dispositif de stimulation (1) ;
- une distribution relative de décalages de phase de N courants alternatifs d'entraînement ou N tensions alternatives d'entraînement à appliquer aux N électrodes de stimulation (2) du dispositif de stimulation (1) ;
- un schéma temporel pour N tensions CA d'entraînement à appliquer à N électrodes de stimulation (2) du dispositif de stimulation (1), incluant de préférence N décalages temporels relatifs spécifiques et/ou une séquence temporelle respective d'activation de chacune des électrodes de stimulation ;
- un ensemble de N formes d'onde de N tensions CA d'entraînement à appliquer à N électrodes de stimulation (2) du dispositif de stimulation (1).

5. Procédé selon l'une quelconque des revendications précédentes, dans lequel les paramètres de configuration comprennent un protocole de stimulation électrique spécifique à un patient pour entraîner un certain nombre de N électrodes de stimulation (2) du dispositif (1) dans un schéma de stimulation transcrânienne à courant alternatif (tACS),
- de préférence dans lequel le protocole de stimulation comprend au moins un, de préférence au moins deux, des paramètres de stimulation électrique suivants :
- un ampérage, de préférence une amplitude de CA, et/ou
- une fréquence et/ou
- un schéma d'entraînement de CA et/ou
- une phase relative et/ou
- une forme d'onde et/ou
- un décalage temporel
de courants d'entraînement individuels à appliquer aux électrodes de stimulation individuelles (2) du dispositif de stimulation (1).

6. Procédé selon la revendication 5, dans lequel l'étape D) comprend la configuration du dispositif de stimulation (1) avec ledit protocole de stimulation électrique.

7. Procédé selon l'une quelconque des revendications précédentes, dans lequel le procédé comprend en outre une étape consistant à E) choisir ledit dispositif de stimulation électrique (1) parmi un ensemble prédéfini de M dispositifs de stimulation différents (1) sur la base de l'ensemble calculé de paramètres de configuration,
- de préférence dans lequel les M dispositifs de stimulation (1) compris dans l'ensemble prédéfini diffèrent :
- dans leur nombre respectif N d'électrodes de stimulation individuelles disponibles (2) et/ou
- dans un agencement géométrique respectif, fixe ou réglable, d'un certain nombre de N électrodes de stimulation (2) et/ou
- dans une forme individuelle respective d'au moins une des électrodes de stimulation respectives (2).

8. Procédé selon l'une quelconque des revendications précédentes, dans lequel les données spécifiques à un patient extraites à l'étape A) comprennent au moins un, de préférence au moins deux, des paramètres spécifiques à un patient suivants :
- des données géométriques d'un crâne du patient, comme une distribution d'une épaisseur de l'os du crâne et/ou du cuir chevelu ;
- une distribution 3D des tissus cérébraux, de préférence la taille du cerveau du patient et/ou la distribution de la matière grise et blanche dans le cerveau du patient ;
- des concentrations localisées de protéines spécifiques, en particulier de protéines amyloïdes et/ou tau, dans le cerveau du patient ;
- l'apparition localisée d'une atrophie dans le cerveau du patient ;
- des coordonnées 3D d'au moins une région d'intérêt (ROI) à électrostimuler à l'aide du dispositif (1) ;
- de préférence dans lequel l'optimisation effectuée à l'étape B) prend en compte au moins l'un des paramètres spécifiques à un patient pour calculer ledit ensemble de paramètres de configuration, et/ou
- dans lequel l'optimisation effectuée à l'étape B) est basée sur un modèle de tête géométrique dérivé des données spécifiques à un patient extraites à l'étape A).

9. Procédé selon l'une quelconque des revendications précédentes, dans lequel les paramètres de simulation modifiés à l'étape C) comprennent au moins un, de préférence au moins deux, des paramètres suivants :
- un nombre N d'électrodes de stimulation disponibles (2) du dispositif (1) ;
- un agencement géométrique de N électrodes de stimulation disponibles (2) du dispositif (1) ;
- une forme respective des électrodes de stimulation disponibles (2) du dispositif (1) ;
- une intensité et/ou fréquence de courants d'entraînement à appliquer aux électrodes de stimulation individuelles (2) du dispositif (1) ;
- un schéma de pilotage électrique c.a./c.c. à appliquer au dispositif (1) ;

10. Procédé selon l'une quelconque des revendications précédentes, dans lequel l'étape C est effectuée pour
- maximiser une intensité de champ électrique local dans au moins une région d'intérêt (ROI) extraite de la au moins une image à l'étape A), de préférence tout en minimisant l'intensité de champ électrique dans les régions tissulaires restantes et/ou
- utiliser un algorithme d'optimisation, de préférence basé sur un procédé par éléments finis, et/ou
- de telle sorte que dans des régions restantes en dehors de la au moins une région d'intérêt (ROI), extraites de la au moins une image à l'étape A), une intensité de champ effective reste en dessous d'un potentiel d'action physiologique (AP) des cellules neuronales présentes dans ces régions restantes.

11. Procédé selon l'une quelconque des revendications précédentes, dans lequel au cours de l'étape C), au moins une, de préférence au moins deux, des conditions limites suivantes sont prises en compte :
- une tension d'entraînement maximale et/ou courant d'entraînement disponible pour entraîner une électrode de stimulation individuelle (2) du dispositif (1) ;
- une plage de fréquence disponible pour un courant d'entraînement à appliquer à une électrode de stimulation individuelle (2) du dispositif (1) ;
- un nombre M d'agencements géométriques fixes disponibles de N électrodes de stimulation (2) fournis par un ensemble limité de M dispositifs de stimulation différents disponibles (1) ;
- un nombre maximal Nₘₐₓ d'électrodes de stimulation disponibles (2) ;
- un nombre limité W de différentes formes disponibles d'électrodes de stimulation (2).

12. Procédé selon l'une quelconque des revendications précédentes, dans lequel la au moins une image préexistante (3) comprend au moins un parmi, de préférence au moins deux parmi,
- une image de tomographie par résonance magnétique (TRM) (3) et/ou
- une image de tomographie par émission de positons (TEP) (3) et/ou
- une image de tomodensitométrie (CT) à rayons X (3) et/ou
- dans lequel l'étape A) comprend l'extraction de concentrations localisées de protéines particulières, définissant des régions d'intérêt respectives (ROI), à partir d'images de TEP préexistantes (3), de préférence dans lequel l'étape A) comprend des images de TEP (3) acquises à l'aide de marqueurs spécifiques à une protéine, de préférence radioactifs.

13. Procédé selon l'une quelconque des revendications précédentes, dans lequel le dispositif de stimulation (1) présente au moins une électrode, de préférence l'une des N électrodes de stimulation (2), configurée ou configurable pour l'enregistrement
- de champs électriques générés avec certaines des N électrodes de stimulation (2) et/ou
- de signaux nerveux provenant du tissu neuronal, de préférence des ondes cérébrales,
- de préférence dans lequel des champs électriques et/ou des signaux nerveux précédemment enregistrés avec le dispositif de stimulation (1) sont utilisés comme entrée pour optimiser les paramètres de configuration au cours des étapes B) et C).

14. Procédé selon l'une quelconque des revendications précédentes, dans lequel le dispositif de stimulation (1) présente
- un nombre de N électrodes de stimulation non invasives (2) agencées selon une géométrie 3D,
- un circuit de commande électronique (4) pour fournir des tensions ou des courants d'entraînements électriques individuels aux électrodes de stimulation individuelles (2),
- une interface de données (5) pour configurer ledit dispositif de stimulation (1) avec un ensemble de paramètres de configuration spécifique à un patient,
- dans lequel les N électrodes de stimulation (2) sont agencées sur une coque de forme stable (20) d'un casque (19) ou d'un capot (9) avec une conception spécifique à un patient qui est basée sur l'anatomie du cerveau d'un patient individuel et qui est de forme stable,
- et dans lequel le dispositif de stimulation (1) est configuré avec un ensemble de paramètres de configuration qui ont été dérivés à l'aide d'un procédé selon l'une quelconque des revendications 1 à 13,
- en particulier dans lequel l'agencement géométrique des N électrodes de stimulation (2) est reconfiguré.

15. Procédé de calcul d'une série d'ensembles individuels de paramètres de configuration optimisés,
- dans lequel chaque ensemble est calculé en mettant en oeuvre un procédé selon l'une quelconque des revendications précédentes 1 à 14,
- de préférence dans lequel chacun des ensembles individuels est destiné à configurer le dispositif de stimulation (1) avant une session de thérapie spécifique d'une série de telles sessions, et/ou
- dans lequel chacun des ensembles est optimisé pour permettre la génération d'un motif d'électrostimulation prédéfini à l'aide du dispositif de stimulation (1),
- de préférence dans lequel les différents modèles d'électrostimulation consécutifs définissent un schéma d'électrothérapie spécifique.

16. Programme informatique, de préférence conçu sous la forme d'une application à exécuter sur un dispositif électronique mobile (7), comprenant des instructions qui, lorsque le programme est exécuté par un ordinateur/ledit dispositif électronique (7), amènent l'ordinateur/ledit dispositif électronique (7) à exécuter un procédé selon l'une des revendications 1 à 14.
